# EUROPÄISCHE PATENTSCHRIFT

(11) **EP 1 471 841 B1**
(45) Veröffentlichungstag und Bekanntmachung des Hinweises auf die Patenterteilung: **04.06.2008**
(21) Anmeldenummer: 02716032.4
(22) Anmeldetag: 07.02.2002
(51) Int. Cl.: A61B 17/58, A61B 5/107

(54) **VORRICHTUNG ZUR LÄNGEN- UND TIEFENMESSUNG IN DER CHIRURGIE**
DEVICE FOR MEASURING LENGTH AND DEPTH WHICH CAN BE USED IN SURGERY
DISPOSITIF DE MESURE DE LONGUEUR ET DE PROFONDEUR UTILISE EN CHIRURGIE

(43) Veröffentlichungstag der Anmeldung: 03.11.2004
(73) Patentinhaber: Synthes GmbH, 4436 Oberdorf (CH)
(72) Erfinder: RUPP, Stephan, CH-7240 Küblis (CH); PELTZER, Jörg, 2800 Delémont (CH)
(74) Vertreter: Lusuardi, Werther
(86) Internationale Anmeldenummer: PCT/CH2002/000071
(87) Internationale Veröffentlichungsnummer: WO 2003/065910

(56) Entgegenhaltungen:
- US-A- 5 013 318
- US-A- 5 242 448
- US-A- 5 928 243

## Beschreibung

Die Erfindung bezieht sich auf eine Vorrichtung zur Längen- und Tiefenmessung in der Chirurgie, gemäss dem Oberbegriff des Patentanspruchs 1.

Bei osteosynthetischen Behandlungen, beispielsweise von Knochenfrakturen ist der Einsatz von Knochenschrauben häufig. Die Knochenschrauben werden vielfach senkrecht zur Knochenlängsachse in den Knochen eingeschraubt. Dabei dringt die Knochenschraube in die Gegenkortikalis ein. Die Spitze der Schraube sollte aber nicht zu weit in den Weichteilmantel hinausragen. Weichteilirritationen oder Verletzungen können die Folge sein. Daher ist die exakte Längenbestimmung für die in ein vorgebohrtes Schraubenloch einzusetzende Knochenschraube wichtig. Mit den bisher bekannten Längen- oder Tiefenmessgeräten wird ein Kolben mit einem Haken durch die Bohrung eingeführt und an der Gegenkortikalis, auf der Weichteilseite, eingehakt. Anschliessend wird die Messhülse auf dem Kolben so lange in Richtung der Knochenoberfläche verschoben, bis das vordere Ende der Messhülse an der Knochenoberfläche ansteht. Dabei besteht jedoch die Gefahr, dass der Haken an der Gegenkortikalis abrutscht und in der Folge der Haken durch das Verschieben der Messhülse in den Knochen gezogen wird. Dies kann dann zu fehlerhaften Längenmessungen und in der Folge zur falschen Längenwahl der Knochenschraube führen.

Aus der US-A-5 013 318 SPRANZA III ist eine Vorrichtung zur Längen- und Tiefenmessung in einem Knochen bekannt, bei welcher eine Anzahl Drähte, deren Spitzen abgewinkelt sind, aus einer Hülse drehbar sind. Bei diesem bekannten Instrument liegen die Drähte offen am Instrument auf und branchen einen gewissen Raum, um die Drähte aus der Hülse herausfahren zu können.

Aus der US 5,242,448 PETTINE ET AL. ist eine Knochensonde bekannt, bei welcher ein Draht in einer Bohrung mit distaler Krümmung verschieblich geführt wird. Bei diesem bekannten Gerät fehlen Feststellmittel.

Die Längenmessung erfolgt durch einfache Längsverschiebung des Drahtes.

Hier will die Erfindung Abhilfe schaffen. Der Erfindung liegt die Aufgabe zugrunde, ein Längen- oder Tiefenmessgerät zu schaffen, bei welchem nach dem Einfahren des Kolbens in die Bohrung über die Gegenkortikalis hinaus ein Haken ausgefahren werden kann und so eine formschlüssige Verbindung erreichbar ist und zudem die Länge nicht in-situ abgelesen werden muss.

Die Erfindung löst die gestellte Aufgabe mit einer Vorrichtung zur Längen- und Tiefenmessung in der Chirurgie, welche die Merkmale des Anspruchs 1 aufweist.

Die erfindungsgemässe Vorrichtung umfasst zwei koaxial zu einer Längsachse angeordneten, teleskopierbaren Teilen. Dabei ist das erste Teil als Messkolben ausgeführt und das zweite Teil als Messhülse ausgeführt. Der Messkolben ist axial in der Zentralbohrung der Messhülse verschiebbar und mit seinem Vorderteil in eine Bohrung in einem Knochen einführbar. Der Messkolben hat am Vorderteil eine Spitze und umfasst bewegbare Arretiermittel, welche im Messkolben bewegbar angeordnet sind und einen Haken umfassen, der an der Spitze quer zur Längsachse aus- und einfahrbar ist.

Zum Messen wird der Messkolben mit zurückgeschobener Messhülse in die Bohrung eingeführt und soweit durch die Bohrung durchgeschoben, bis die Spitze über die Gegenkortikalis hinausragt. Anschliessend wird der Haken bei der Spitze aus dem Vorderteil ausgefahren. Mit ausgefahrenem Haken kann der Messkolben nicht mehr aus der Bohrung herausgezogen werden und die gesamte Vorrichtung nicht mehr vom Knochen zurückgezogen werden. Der darauf folgende Messvorgang umfasst das Verschieben der Messhülse auf dem Messkolben in Richtung der Knochenoberfläche bis das vordere Ende der Messhülse auf der Knochenoberfläche zur Anlage kommt. Dabei wird der Knochen zwischen dem ausgefahrenen Haken und der Messhülse eingeklemmt. Nach dem Messvorgang wird der Haken wieder quer zur Längsachse in den Messkolben eingefahren, die Vorrichtung aus der Bohrung im Knochen herausgezogen und aus dem Operationsfeld entfernt.

Die durch die Erfindung erreichten Vorteile sind im wesentlichen darin zu sehen, dass dank der erfindungsgemässen Vorrichtung: .
- bei ausgefahrenem Haken kein Abrutschen des Messkolbens an der Gegenkortikalis erfolgen kann; und
- das Ablesen der zu messenden Länge nicht in-situ erfolgen muss und dadurch eine gute Ablesbarkeit erreichbar ist.

In einer bevorzugten Ausführungsform der erfindungsgemässen Vorrichtung sind die Messhülse und der Messkolben mit Feststellmitteln ausgestattet. Durch diese Feststellmittel wird der Messkolben relativ zur Messhülse in einer gewählten Position gehalten, so dass die Vorrichtung vom Knochen entfernbar ist und die zu messende Länge ausserhalb des Operationsfeldes ablesbar ist. Dies ermöglicht eine gute Ablesbarkeit der zu messenden Länge ausserhalb des Operationsfeldes. Die Vorrichtung kann vom Operateur frei gehalten werden und Lichtreflexe oder die Schriftart der Markierung haben keinen störenden Einfluss.

Die dadurch erreichten Vorteile sind im wesentlichen darin zu sehen, dass:
- durch die zwischen Messkolben und Messhülse angeordneten Feststellmittel ein unbeabsichtigtes Verschieben von Messkolben und Messhülse relativ zueinander ausgeschlossen wird.

In der erfindungsgemässen Vorrichtung umfassen die Arretiermittel eine Schraube, welche parallel zur Längsachse im ersten Teil ein- respektive ausschraubbar ist. Koaxial zur Längsachse ist am vorderen Ende der Schraube axial fest aber um die Längsachse rotierbar ein Stab mit einem Draht angebracht, wobei auch hier der Draht je nach der axialen Position der Schraube den aus- respektive eingefahrenen Haken bildet.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Teilschnitt durch eine Ausführungsform der erfindungsgemässen Vorrichtung;
Fig. 2 einen Schnitt durch die erfindungsgemässe Vorrichtung;
Fig. 3 einen Teilschnitt durch eine andere Ausführungsform der erfindungsgemässen Vorrichtung;
Fig. 4 einen Teilschnitt durch wiederum eine andere Ausführungsform der erfindungsgemässen Vorrichtung; und
Fig. 5 einen Teilschnitt durch nochmals eine weitere Ausführungsform der erfindungsgemässen Vorrichtung.

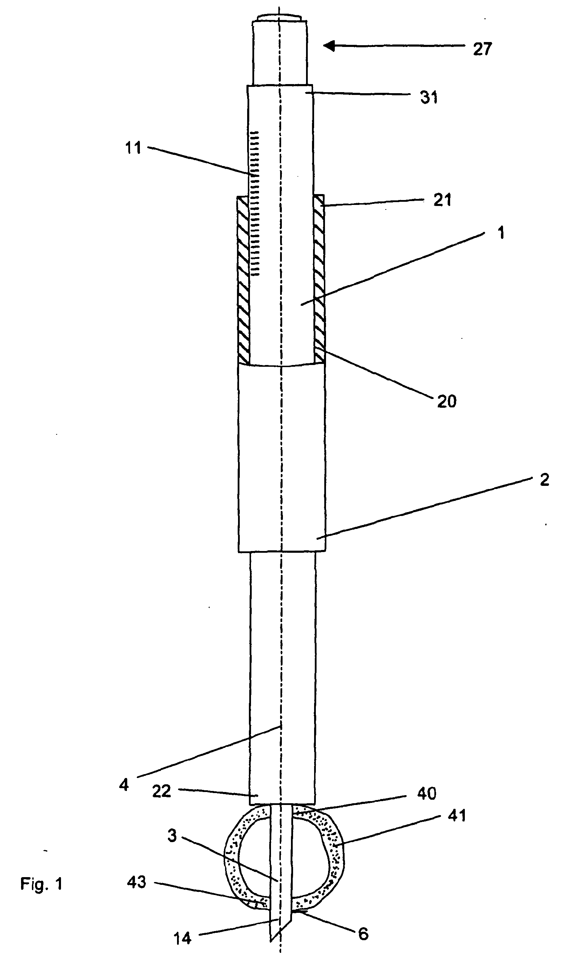

Fig. 1 zeigt eine Ausführungsform der erfindungsgemässen Vorrichtung mit einem ersten Teil 1 und einem zweiten Teil 2, wobei das erste Teil 1 als Messkolben und das zweite Teil 2 als Messhülse ausgeführt sind. Das erste Teil 1 ist parallel zur Längsachse 4 innerhalb der Zentralbohrung 20 im zweiten Teil 2 verschiebbar. Über das vordere Ende 22 des zweiten Teiles 2 ragt je nach relativer Position zwischen dem ersten Teil 1 und dem zweiten Teil 2 das Vorderteil 3 des ersten Teiles 1 mit der Spitze 14 hinaus. Das Vorderteil 3 ist soweit in eine Bohrung 40 in einem Knochen 41 einführbar bis die Spitze 14 über die Gegenkortikalis 43 des Knochens 41 hinausragt. Innerhalb des ersten Teiles 1 sind die Arretiermittel 27 angeordnet. Verbunden mit den Arretiermitteln 27 ist ein Haken 6, welcher bei der Spitze 14 quer zur Längsachse 4 aus dem Vorderteil 3 aus- respektive eingefahren wird, so dass das Vorderteil 3 nicht mehr aus der Bohrung 40 im Knochen 41 herausgezogen werden kann. Ferner ist am ersten Teil 1 eine Skala 11 angebracht, so dass am hinteren Ende 21 des zweiten Teiles 2 auf der Skala 11 die zu ermittelnde Länge abgelesen werden kann.

Wenn die erfindungsgemässe Vorrichtung zu einer Längen- oder Tiefenmessung vorbereitet wird, ist das zweite Teil 2 in seiner hintersten Position, d.h. das hintere Ende 21 des zweiten Teiles 2 ist so nahe wie möglich beim hinteren Ende 31 des ersten Teiles 1. Als Anschlag für diese hinterste Position dient ein Absatz 37 in der Zentralbohrung 20 des zweiten Teiles 2, woran die Hülse 28 und das hintere Ende 15 des Vorderteiles 3 zur Anlage kommen.

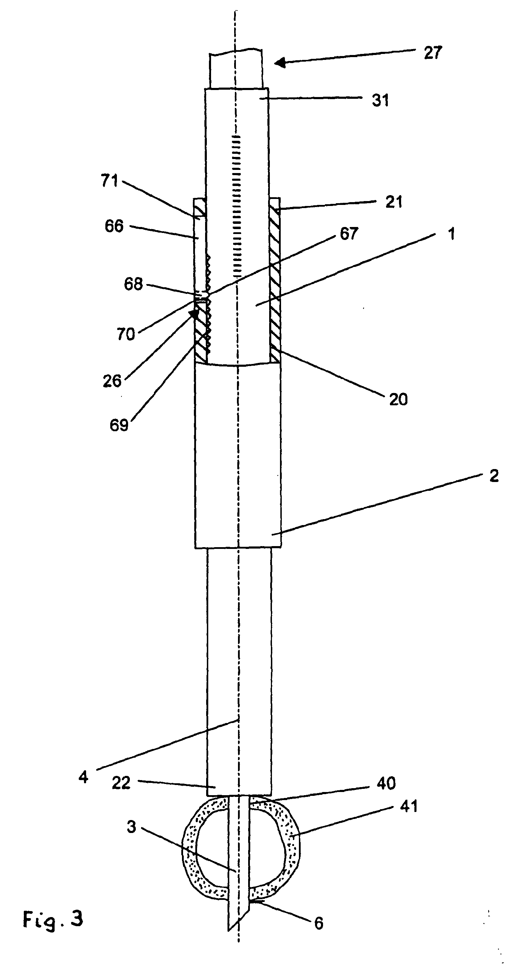

In Fig. 1 dargestellt ist eine Anwendung beispielsweise bei der Längenmessung einer in einen Knochen 41 einzudrehenden Knochenschraube. Zur Durchführung der Längenmessung wird das Vorderteil 3 des ersten Teiles 1 in die Bohrung 40 im Knochen 41 eingeführt bis die Spitze 14 über die Gegenkortikalis 43 hinausragt. Das zweite Teil 2 wird dazu mit seinem hinteren Ende 21 so nahe wie möglich an das hintere Ende 31 des ersten Teiles 1, d.h. in seine hinterste Position verschoben. Im nächsten Schritt wird der Haken 6 des Arretiermittels 27 mittels der am hinteren Ende 31 des ersten Teiles 1 angeordneten Bedienungsmittel ausgefahren. Anschliessend wird das zweite Teil 2 nach vorne, d.h. gegen das Vorderteil 3 verschoben bis das vordere Ende 22 des zweiten Teiles 2 an der Oberfläche des Knochens 41 ansteht (Fig. 1). Dabei wird der Knochen 41 zwischen dem vorderen Ende 22 des zweiten Teiles 2 und dem Haken 6 eingeklemmt. Durch die Feststellmittel 26 (Fig. 3 bis 5) wird das zweite Teil 2 relativ zum ersten Teil 1 in seiner Position gehalten. Im nächsten Schritt wird nun der Haken 6 des Arretiermittels 27 durch entsprechende Handhabung der Bedienungsmittel eingefahren und die Vorrichtung aus der Bohrung 40 im Knochen 41 herausgezogen. Die gemessene Länge kann anschliessend im Operationsraum ausserhalb des Operationsfeldes auf einer Skala 11 (Fig. 1) abgelesen werden.

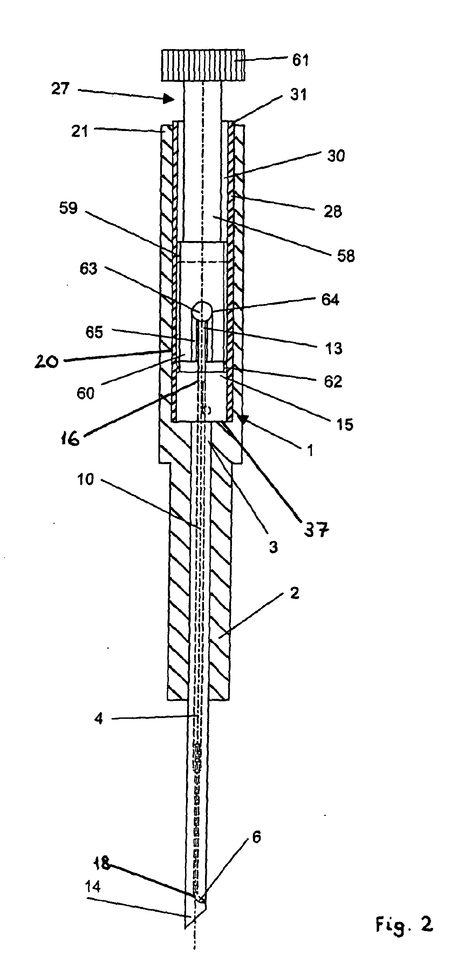

Die in Fig. 2 dargestellte erfindungsgemässe Vorrichtung, zeigt das erste Teil 1 mit dem Vorderteil 3 und einer Hülse 28, welche mit dem hinteren Ende 15 des Vorderteiles 3 verbunden ist und bis zum hinteren Ende 31 des ersten Teiles 1 reicht. Das Vorderteil 3 ist vom hinteren Ende 15 bis zur Spitze 14 durchbohrt, wobei diese Bohrung 16 an der Spitze 14 mittels einer Krümmung 18 oder Abwinkelung abgewinkelt oder gekrümmt ist, so dass der Austritt der Bohrung 16 an der Spitze 14 quer zur Längsachse 4 gerichtet ist, und von dort bis zum hinteren Ende 15 bis zur Spitze 14 koaxial zur Längsachse 4 verläuft.

In der in Fig. 2 dargestellten Ausführungsform umfassen die Arretiermittel 27 eine Schraube 58. Die Schraube 58 ist an ihrem hinteren Ende 61, welches über das hintere Ende 31 des ersten Teiles 1 hinausragt, von Hand drehbar. Auf ihrem vorderen Teil bis zu ihrem vorderen Ende 60 ist die Schraube 58 mit einem Aussengewinde 59 versehen. Das Aussengewinde 59 ist in ein Innengewinde 62 im ersten Teil 1 schraubbar, wobei das Innengewinde 62 in der Zentralbohrung 30 in der Hülse 28 angebracht ist und bis zum hinteren Ende 15 des Vorderteils 3 reicht. Somit ist die Schraube 58 in das erste Teil 1 soweit einschraubbar, bis das vordere Ende 60 der Schraube 58 am hinteren Ende 15 des Vorderteils 3 ansteht. Falls die Schraube 58 in ihrer hinteren Position ist, ist der Haken 6 in seiner eingefahrenen Position. Andernfalls, wenn die Schraube 58 in ihre vordere Position gebracht wird, d.h. so weit in das erste Teil 1 eingeschraubt wird, bis das vordere Ende 60 der Schraube 58 am hinteren Ende 15 des Vorderteils 3 ansteht, ist der Haken 6 in seiner ausgefahrenen Position festgestellt, wobei die Arretierung des Hakens 6 in dieser Position durch die Gewindeverbindung zwischen dem ersten Teil 1 und der Schraube 58 erfolgt. Der Stab 10 weist an seinem hinteren Ende 13 einen kugelförmigen Kopf 63 auf. Das hintere Ende 13 mit dem kugelförmigen Kopf 63 wird in einem Sackloch 64, welches in der Schraube 58 angebracht ist, aufgenommen. Das Sackloch 64 verläuft koaxial zur Längsachse 4 und ist am vorderen Ende 60 der Schraube 58 offen. Der kugelförmige Kopf 63 wird gegen das vordere Ende 60 der Schraube 58 beispielsweise durch einen Einsatz 65 im Sackloch 64 axial festgehalten. Somit ist der Stab 10 axial fixiert aber um die Längsachse 4 rotierbar mit der Schraube 58 verbunden.

In den Fig. 3 bis 5 werden Ausführungsformen der erfindungsgemässen Vorrichtung gemäss einer der Ausführungsformen nach Fig. 1 oder 2 mit Feststellmitteln 26 zur lösbaren Blockierung der relativen axialen Position zwischen dem ersten Teil 1 und dem zweiten Teil 2 dargestellt.

Fig. 3 zeigt eine Ausführungsform der erfindungsgemässen Vorrichtung, bei welcher die Feststellmittel 26 eine federnde Zunge 66, welche quer zur Längsachse 4 federbar ist, und eine Verzahnung 69 umfassen, welche am ersten Teil 1 parallel zur Längsachse 4 angebracht ist. Die federnde Zunge 66 ist mit ihrem festen Ende 71 am zweiten Teil 2 in der Nähe des hinteren Endes 21 des zweiten Teiles 2 befestigt und ist an ihrem freien Ende 70 mit einer gegen die Zentralbohrung 20 des zweiten Teiles 2 gerichteten Erhebung 67 versehen. Die Erhebung 67 greift in die Verzahung 69 ein, so dass dadurch eine axiale Fixierung des ersten Teiles 1 relativ zum zweiten Teil 2 erreichbar ist. Die Verzahnung 69 kann beispielsweise Zähne in Millimeterschritten aufweisen, so dass das erste Teil 1 in Millimeterschritten im zweiten Teil 2 feststellbar ist. Die Erhebung 67 kann beispielsweise durch einen Stift 68 ausgebildet werden, dessen Spitze in die Verzahnung 69 eingreift.

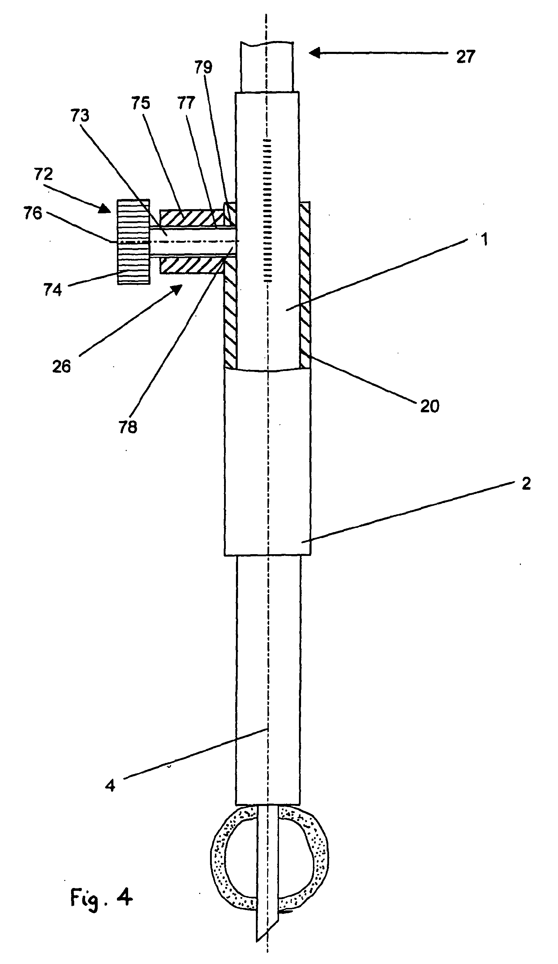

Fig. 4 zeigt eine Ausführungsform der erfindungsgemässen Vorrichtung, bei welcher die Feststellmittel 26 eine Fixierschraube 72 mit einer quer zur Längsachse 4 verlaufenden Schraubenachse 76 umfassen. Der Schraubenschaft 73 dieser Fixierschraube 72 ist durch eine Bohrung 79 im zweiten Teil 2 durchführbar und in das Innengewinde 77 einer Büchse 75 einschraubbar, so dass das vordere Ende 78 der Fixierschraube 72 in die Zentralbohrung 20 des zweiten Teiles 2 reicht und gegen das erste Teil 1 pressbar ist. Die Büchse 75 ist am zweiten Teil 2 befestigt. Der Schraubenkopf 74 der Fixierschraube 72 steht entgegengesetzt über die Büchse 75 vor und ist von Hand drehbar, so dass die Blockierung zwischen dem ersten Teil 1 und dem zweiten Teil 2 durch Anziehen erreichbar ist. Das Innengewinde 77 kann bei ausreichender Wandstärke zwischen Aussenfläche und Zentralbohrung 20 am zweiten Teil 2 auch in der Bohrung 79 am zweiten Teil 2 angebracht sein.

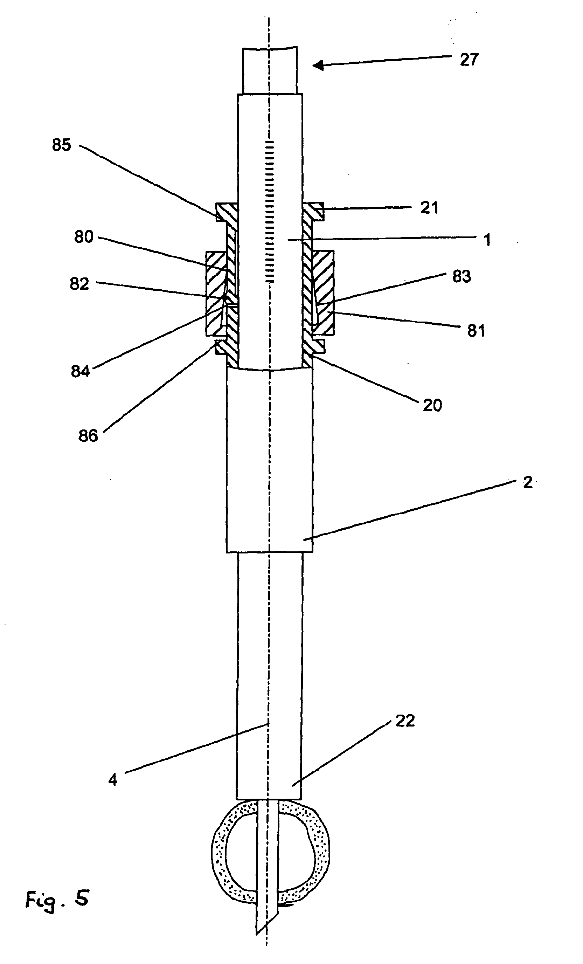

Fig. 5 zeigt eine Ausführungsform der erfindungsgemässen Vorrichtung, bei welcher die Feststellmittel 26 eine Lasche 80 umfassen, welche quer zur Längsachse 4 elastisch deformierbar ist und mit dem zweiten Teil 2 verbunden ist. Die Lasche 80 ist mittels einer Klemmhülse 81, welche parallel zur Längsachse 4 auf dem zweiten Teil 2 verschiebbar ist, seitlich gegen das erste Teil 1 pressbar, so dass das erste Teil 1 relativ zum zweiten Teil 2 fixierbar ist. Zum Lösen oder Anziehen der Feststellmittel 26 hat die Lasche 80 an ihrem elastisch deformierbaren Ende 84 eine Erhebung 82, welche an die Wand des in der Klemmhülse 81 angebrachten, sich gegen das hintere Ende 21 des zweiten Teiles 2 verjüngenden Innenkonus 83 grenzt. Wird die Klemmhülse 81 gegen das vordere Ende 22 des zweiten Teiles 2 verschoben, presst der Innenkonus 83 auf die Erhebung 82, wodurch die Lasche 80 seitlich gegen das erste Teil 1 gepresst wird. Zum Lösen der Feststellmittel 26 wird die Klemmhülse 81 axial gegen das hintere Ende 21 des zweiten Teiles 2 verschoben, bis der Innenkonus 83 die Erhebung 82 frei gibt. Die axiale Verschiebung der Klemmhülse 81 wird gegen das hintere Ende 21 des zweiten Teiles 2 durch einen ersten Anschlag 85 am hinteren Ende 21 des zweiten Teiles 2 und gegen das vordere Ende 22 des zweiten Teiles 2 durch einen zweiten Anschlag 86 begrenzt.

Durch die Feststellmittel 26 wird das zweite Teil 2, nachdem dessen vorderes Ende 22 an der Oberfläche des Knochens 41 zur Anlage gebracht wurde, relativ zum ersten Teil 1 in seiner Position gehalten, so dass nach dem Einfahren des Haken 6 des Arretiermittels 27 die Vorrichtung aus der Bohrung 40 im Knochen 41 herausgezogen werden kann, ohne dass dabei die relative axiale Position zwischen dem ersten Teil 1 und dem zweiten Teil 2 verrutschen kann.

## Patentansprüche

1. Vorrichtung zur Längen- und Tiefenmessung in der Chirurgie, umfassend
A) zwei koaxial zu einer Längsachse (4) angeordnete, longitudinale und teleskopierbare Teile (1;2), wobei
B) das zweite Teil (2) ein hinteres Ende (21), ein vorderes Ende (22) und eine das zweite Teil (2) koaxial durchdringende Zentralbohrung (20) umfasst und das vordere Ende (22) an der Oberfläche eines Knochens zur Anlage bringbar ist; und
C) das erste Teil (1) in der Zentralbohrung (20) des zweiten Teiles (2) koaxial zur Längsachse (4) verschiebbar ist, ein Vorderteil (3) mit einer Spitze (14) und ein hinteres Ende (31) umfasst und das Vorderteil (3) des ersten Teils (1) in eine Bohrung (40) in einem Knochen (41) einführbar ist, so dass die Spitze (14) über die Gegenkortikalis (43) hinausragt, wobei
D) das erste Teil (1) bewegbare Arretiermittel (27) mit einem an der Spitze (14) des Vorderteils (3) quer zur Längsachse (4) aus- respektive einfahrbaren Haken (6) umfasst,
**dadurch gekennzeichnet, dass**
E) das erste Teil (1) von seinem hinteren Ende (31) bis zur Spitze (14) eine zentrale Bohrung (16) umfasst, welche im Vorderteil (3) bei der Spitze (14) mittels einer Krümmung (18) oder Abwinkelung abgewinkelt oder gekrümmt ist, so dass der Austritt der Bohrung (16) an der Spitze (14) quer zur Längsachse (4) gerichtet ist; und
F) die Arretiermittel (27) folgendes umfassen
f1) eine Schraube (58), welche parallel zur Längsachse (4) im ersten Teil (1) einrespektive ausschraubbar ist und ein vorderes Ende (60) hat;
f2) einen am vorderen Ende (60) der Schraube (58) bezüglich der Längsachse (4) axial festen aber um diese rotierbaren Stab (10) mit einem der Schraube (58) axial entgegengesetzten vorderen Ende (12); und
f3) einen axial fest mit dem vorderen Ende (12) des Stabes (10) verbundenen Draht (17), welcher in der Bohrung (16) verschiebbar ist und durch die Krümmung (18) oder Abwinkelung in einen zur Längsachse (4) koaxialen Teil und in einen quer zur Längsachse (4) verlaufenden, den Haken (6) bildenden Teil bringbar ist.

2. Vorrichtung nach Anspruch 1, **dadurch gekennzeichnet, dass** sie Feststellmittel (26) umfasst, wodurch das erste Teil (1) in seiner axialen Position relativ zum zweiten Teil (2) lösbar fixierbar ist.

3. Vorrichtung nach Anspruch 2, **dadurch gekennzeichnet, dass** die Feststellmittel (26)
A) eine am zweiten Teil (2) angebrachte federnde Zunge (66) mit einem freien, quer zur Längsachse (4) federbaren Ende (70) und einer gegen die Zentralbohrung (20) im zweiten Teil (2) gerichteten Erhebung (67) am freien Ende (70); und
B) eine am ersten Teil (1) parallel zur Längsachse (4) verlaufende Verzahnung (69) umfassen, wobei
C) die Erhebung (67) in die Verzahnung (69) in Eingriff bringbar ist.

4. Vorrichtung nach Anspruch 2, **dadurch gekennzeichnet, dass** die Feststellmittel (26)
A) eine Fixierschraube (72) mit einer quer zur Längsachse (4) verlaufenden Schraubenachse (76), einem Schraubenkopf (74), einem vorderen Ende (79) und einem an das vordere Ende (79) angrenzenden Schraubenschaft (73); und
B) eine zur Schraubenachse (76) koaxiale Bohrung (79) im zweiten Teil (2) umfassen, worin der Schraubenschaft (73) durchführbar ist, so dass das vordere Ende (79) der Fixierschraube (72) bis in die Zentralbohrung (20) im zweiten Teil (2) reicht und seitlich auf das erste Teil (1) pressbar ist.

5. Vorrichtung nach Anspruch 2, **dadurch gekennzeichnet, dass** die Feststellmittel (26)
A) eine am zweiten Teil (2) befestigte, quer zur Längsachse (4) elastisch deformierbare Lasche (80) mit einer quer zur Längsachse (4) vorstehenden Erhebung (82); und
B) eine parallel zur Längsachse (4) auf dem zweiten Teil (2) verschiebbare Klemmhülse (81) mit einem sich gegen das hintere Ende (21) des zweiten Teiles (2) verjüngenden Innenkonus (83) umfassen, wobei
C) bei axialer Verschiebung der Klemmhülse (81) gegen das vordere Ende (22) des zweiten Teiles (2) der Innenkonus (83) auf die Erhebung (82) drückt, so dass dadurch die Lasche (80) in die Zentralbohrung (20) des zweiten Teiles (2) gedrückt und das erste Teil (1) relativ zum zweiten Teil (2) fixiert wird.

## Claims

1. A device for measuring length and depth in surgery comprising:
A) two longitudinal telescopable members (1,2) arranged coaxially to a longitudinal axis (4), whereby
B) the second member (2) comprises a rear end (21), a front end (22) and a central bore (20) coaxially penetrating the second member (2) and whereby the front end (22) can be brought in contact with the surface of a bone; and
C) the first member (1) is displaceable in the central bore (20) of the second member (2) coaxially to the longitudinal axis (4) and comprising a front part (3) with a tip (14) and a rear end (31), and the front part (3) of the first member (1) is insertable in a bore hole (40) in a bone (41) such that the tip (14) projects over the opposite corticalis (43), whereby
D) the first member (1) comprises displaceable stopping means (27) with a hook (6) which is telescopable respectively retractable transversely to the longitudinal axis (4) at the tip (14) of the front part (3),
**characterized in that**
E) the first member (1) comprises a central bore (16) from its rear end (31) to the tip (14), which is angled or curved by means of a curvature (18) or a bend in the front part (3) at the tip (14) such that the opening of the bore (16) is directed transversely to the longitudinal axis (4) at the tip (14); and
F) the stopping means (27) comprise
f1) a screw (58), which can be screwed into or out of the first part (1) parallel to the longitudinal axis (4) and has a front end (60),
f2) a rod (10), which is fixed at the front end (60) of the screw (58) with respect to the longitudinal axis (4), but can be rotated about the latter, with a front end (12) axially opposite to the screw (58); and
f3) a wire (17), which is connected axially firmly with the front end (12) of the rod (10) and which is displaceable in the bore (16) and which can be brought in a part extending coaxially to the longitudinal axis (4) and a part, which forms the hook (6) and extends transversely to the longitudinal axis (4) by means of the curvature (18) or bend.

2. The device of claim 1, **characterized in that** it comprises locking means (26), by means of which the first part (1) can be reversibly fixed in its axial position relative to the second part (2).

3. The device of claim 2, **characterized in that** the locking means (26) comprise
A) a flexible flap (66), mounted at the second part (2), with a free end (70), which elastically can yield transversely to the longitudinal axis (4), and an elevation (67) at the free end (70), which is directed towards the central borehole (20) in the second part 2; and
B) a denticulation (69), which extends parallel to the longitudinal axis (4) at the first part (1),
C) it being possible to bring the elevation (67) into engagement with the denticulation (69).

4. The device of claim 2, **characterized in that** the locking means (26) comprise
A) a fixing screw (72) with a screw axis (76) extending transversely to the longitudinal axis (4), a screw head (74), a front end (79) and a screw shaft (73) adjoining the front end (79), and
B) a borehole (79) in the second part (2), which extends coaxially with the screw axis (76) and through which the screw shaft (73) can be passed, so that the front end (79) of the fixing screw (72) extends into the central borehole (20) and can be pressed laterally onto the first part (1).

5. The device of claim 2, **characterized in that** the locking means (26) comprise
A) a flap (80) fixed to the second part (2) and which can be deformed elastically transversely to the longitudinal axis (4), with an elevation (82) protruding transversely to the longitudinal axis (4) and
B) a clamping sleeve (81), which can be shifted on the second part (2) parallel to the longitudinal axis (4), with an internal cone (83), which tapers towards the rear end (21) of the second part (2),
C) when the clamping sleeve (81) is shifted towards the front end (22) of the second part (2), the internal cone (83) being pressed onto the elevation (82), so that, by these means, the flap (80) is pressed into the central borehole (20) of the second part (2) and the first part (1) is fixed relative to the second part (2).

## Revendications

1. Dispositif de mesure de longueur et de profondeur en chirurgie, comprenant :
A) deux éléments longitudinaux et télescopiques (1 ; 2) disposés coaxialement à un axe longitudinal (4), dans lequel
B) le deuxième élément (2) présente une extrémité arrière (21), une extrémité avant (22) et un alésage central (20) traversant coaxialement le deuxième élément (2), et l'extrémité avant (22) peut être mise en appui sur la surface d'un os ; et
C) le premier élément (1) peut être déplacé dans l'alésage central (20) du deuxième élément (2) coaxialement à l'axe longitudinal (4), il comprend une partie avant (3) pourvue d'une pointe (14) et une extrémité arrière (31), et la partie avant (3) du premier élément (1) peut être insérée dans un alésage (40) pratiqué dans un os (41), de sorte que la pointe (14) dépasse de la corticale opposée (43), dans lequel
D) le premier élément (1) comprend des moyens d'arrêt (27) mobiles présentant un crochet (6) rétractable ou déployable transversalement à l'axe longitudinal (4), situé au niveau de la pointe (14) de la partie avant (3),
**caractérisé en ce que**
E) le premier élément (1) comprend un alésage central (16), qui s'étend de son extrémité arrière (31) à la pointe (14), lequel alésage est courbé ou incliné, dans la partie avant (3) au niveau de la pointe (14), au moyen d'une courbure (18) ou d'une inclinaison, de sorte que la sortie de l'alésage (16) au niveau de la pointe (14) est orientée transversalement à l'axe longitudinal (4) ; et
F) les moyens d'arrêt (27) comprennent :
f1) une vis (58), qui peut être vissée ou dévissée parallèlement à l'axe longitudinal (4) dans le premier élément (1) et qui présente une extrémité avant (60) ;
f2) une tige (10) fixe sur l'axe par rapport à l'axe longitudinal (4) mais pouvant tourner autour de celui-ci, située au niveau de l'extrémité avant (60) de la vis (58), dont l'extrémité avant (12) est axialement opposée à la vis (58) ; et
f3) un fil (17), relié de manière fixe sur l'axe à l'extrémité avant (12) de la tige (10), qui peut être glissé dans l'alésage (16) et qui peut être amené, à travers la courbure (18) ou l'inclinaison, dans une partie coaxiale à l'axe longitudinal (4) et dans une partie s'étendant transversalement à l'axe longitudinal (4) qui forme le crochet (6).

2. Dispositif selon la revendication 1, **caractérisé en ce qu'**il comprend des moyens de blocage (26) qui permettent de bloquer de manière amovible le premier élément (1) dans sa position axiale par rapport au deuxième élément (2).

3. Dispositif selon la revendication 2, **caractérisé en ce que** les moyens de blocage (26) comprennent
A) une languette élastique (66), disposée au niveau du deuxième élément (2), qui présente une extrémité (70) libre, pouvant être déformée de manière élastique transversalement à l'axe longitudinal (4), et une protubérance (67), dirigée vers l'alésage central (20) dans le deuxième élément (2), qui est située au niveau de l'extrémité libre (70) ; et
B) une denture (69) s'étendant parallèlement à l'axe longitudinal (4) au niveau du premier élément (1), dans lequel
C) la protubérance (67) peut s'engrener dans la denture (69).

4. Dispositif selon la revendication 2, **caractérisé en ce que** les moyens de blocage (26) comprennent
A) une vis de blocage (72) avec un axe de vis (76) s'étendant transversalement à l'axe longitudinal (4), une tête de vis (74), une extrémité avant (79) et une tige de vis (73) contiguë à l'extrémité avant (79) ; et
B) un alésage (79) coaxial à l'axe de vis (76) dans le deuxième élément (2),
dans lequel la tige de vis (73) peut être insérée de sorte que l'extrémité avant (79) de la vis de blocage (72) rentre dans l'alésage central (20) du deuxième élément (2) et puisse être pressée latéralement contre le premier élément (1).

5. Dispositif selon la revendication 2, **caractérisé en ce que** les moyens de blocage (26) comprennent
A) une patte (80) déformable de manière élastique transversalement à l'axe longitudinal (4), fixée au deuxième élément (2), qui présente une protubérance (82) dépassant transversalement à l'axe longitudinal (4) ; et
B) une douille de serrage (81) pouvant coulisser parallèlement à l'axe longitudinal (4) sur le deuxième élément (2), qui présente un cône intérieur (83) rétrécissant vers l'extrémité arrière (21) du deuxième élément (2), dans lequel
C) lors d'un déplacement axial de la douille de serrage (81) vers l'extrémité avant (22) du deuxième élément (2), le cône intérieur (83) appuie sur la protubérance (82), ce qui pousse la patte (80) dans l'alésage central (20) du deuxième élément (2) et bloque le premier élément (1) par rapport au deuxième élément (2).
